(19) Europäisches Patentamt / European Patent Office / Office européen des brevets

(11) **EP 3 220 960 B1**

(12) **EUROPEAN PATENT SPECIFICATION**

(45) Date of publication and mention
of the grant of the patent:
**25.12.2019 Bulletin 2019/52**

(21) Application number: **15860758.0**

(22) Date of filing: **04.02.2015**

(51) Int Cl.:
*A61K 47/50* (2017.01)   *B82B 3/00* (2006.01)
*B82Y 5/00* (2011.01)   *A61K 9/51* (2006.01)
*A61K 9/14* (2006.01)   *A61K 31/704* (2006.01)
*A61K 47/69* (2017.01)

(86) International application number:
**PCT/RU2015/000064**

(87) International publication number:
**WO 2016/080860 (26.05.2016 Gazette 2016/21)**

(54) **METHOD OF BORON NITRIDE NANOPARTICLE FABRICATION FOR ANTITUMOR DRUG DELIVERY**

VERFAHREN ZUR HERSTELLUNG VON BORNITRIDNANOPARTIKELN FÜR ANTITUMORARZNEIMITTELABGABE

PROCÉDÉ DE FABRICATION DE NANOPARTICULES DE NITRURE DE BORE POUR L'ADMINISTRATION DE MÉDICAMENT ANTITUMORAL

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.11.2014 RU 2014146689**

(43) Date of publication of application:
**27.09.2017 Bulletin 2017/39**

(73) Proprietor: **National University of Science and Technology**
**MISiS**
**119049, Moscow (RU)**

(72) Inventors:
 • **SHTANSKY, Dmitry Vladimirovich**
   **Moscow 125047 (RU)**
 • **KOVALSKII, Andrey Mikhailovich**
   **Moscow 129337 (RU)**
 • **MATVEEV, Andrei Trofimovich**
   **Minsk 220141 (BY)**
 • **SUKHORUKOVA, Irina Viktorovna**
   **Moscow 115419 (RU)**
 • **GLOUSHANKOVA, Natalia Aleksandrovna**
   **Moscow 119607 (RU)**
 • **ZHITNYAK, Irina Yur'evna**
   **Kirov**
   **pos. Ganino 610029 (RU)**

(74) Representative: **Friese Goeden Patentanwälte PartGmbB**
**Widenmayerstraße 49**
**80538 München (DE)**

(56) References cited:
   WO-A1-2011/072482   WO-A2-2012/101639
   JP-A- 2004 182 572   US-A1- 2006 127 422
   US-A1- 2011 033 707

 • XIA LI ET AL.: 'Boron nitride nanotubes functionalized with mesoporous silica for intracellular delivery of chemothetapy drugs.' CHEMICAL COMMUNICATIONS vol. 49, no. 66, 01 January 2013, pages 7337 - 7339, XP055443476 DOI: 10.1039/C3CC42743A

**EP 3 220 960 B1**

**Description**

**Field of the Invention.** This invention relates to nanomedical technologies, more specifically, to the development of transporting nanoagents for the delivery of antitumor chemotherapy drugs.

The reduced efficiency of antitumor chemotherapy during the treatment of oncology patients is primarily attributed to the progression of malignant neoplasms and the generation of cells resulting from tumor progression that have the so-called multiple drug resistance caused by the activation of membrane transporting agents that remove antitumor drugs from the cells. Furthermore, a number of drugs used for the therapy of oncological diseases are low hydrophilic ones and have a toxic effect on healthy body tissues. One way to solve these problems is to use nanotransporting agents delivering drugs to tumor cells.

**Prior Art.** Known is a method of obtaining spherical boron nitride nanoparticles by chemical vapor deposition (US 20110033707, published 10.02.2011). This method allows to obtain boron nitride nanoparticles less than 50 nm in diameter. The method includes heating a mixture of an ether, boric acid and nitrogen in an ammonium and argon atmosphere leading to the formation of the reaction product, its further crystallization which results in formation of spherical nanoparticles precursor and final heating of the precursor in an inert gas.

A disadvantage of this method is the small size of the particles and their smooth surface which does not allow loading the drug in amount required for efficient antitumor therapy and limits the attachment of these particles to tumor cells and their penetration into the latter.

Known is a method of obtaining nanoparticles for drug delivery (WO 2014124329, published 14.08.2014).

This method uses spherical magnetic particles of iron or iron oxide including those coated with gold or silicon oxide. For the therapy of oncological diseases the drug is attached to the magnetic particles through the use of a linker, e.g. ether. The particles heat up by the means of external heating or electromagnetic field, causing the intramolecular cyclization of the linker which results in a release of the drug. This method allows controlled release of drugs thus minimizing side effects and increasing the therapeutic efficiency of usual drugs.

A disadvantage of this method is the necessity of using additional external effect for achieving the required therapeutic efficiency. Another drawback of this method is that some forms of magnetic particles may give rise to the toxicity.

Known is a method for manufacturing nano-carriers, complex of anticancer drug and nano-carrier and pharmaceutical composition for the therapy of oncological diseases (US 20140147508, published 29.05.2014).

This invention relates to a nano-carrier for an anticancer drug, comprising of a spherical metallic nanoparticle and a polynucleotide for drug attachment. The method allows providing a therapeutic effect on tumor cells through the controlled release of a drug in the affected area.

A disadvantage of this method is that the nano-carrier do not deliver drug directly into the tumor cells, so the drug release is carried out in the vicinity of the cells. This reduces the efficiency of the method due to the development of multi drug resistance in the tumor cells.

Also known is a method of delivering anticancer drug to a cancer cells with metal nanoparticles (US 20130331764, published 12.12.2013). The antitumor drug delivery is carried out by the pH-sensitive gold particles of 5-15 nm. After entering the body these pH-sensitive metal nanoparticles are dispersed in normal cells, which are neutral or alkaline, and selectively aggregate in cancer cells which have acidic pH. The therapeutic effect of this method is achieved through the chemical effect of the antitumor drug and the photothermal effect of particle aggregates heated by NIR radiation which deeply penetrates into the patient's body.

A disadvantage of this method is the necessity of additional IR irradiation and the absence of therapeutic efficiency of drug penetration without additional external effect on the patient's body. Furthermore, particles aggregate in tumor cells selectively and thus method efficiency cannot be predicted. Also the 5-15 nm particle size is not desirable due to its excessively rapid absorption by the reticuloendothelial system.

As a prototype we selected a method of obtaining boron nitride nanotubes functionalized with mesoporous silica for intracellular delivery of chemotherapy drugs (X. Li, Ch. Zhi, N. Hanagata, M. Yamaguchi, Y. Bando and D. Golberg, Boron Nitride Nanotubes Functionalized with Mesoporous Silica for Intracellular Delivery of Chemotherapy Drugs, Chem. Commun., 2013, 49, 7337). Method implies obtaining boron nitride nanotubes by chemical vapor deposition, their oxidation in air for 5 h, formation of a mesoporous silica coating by means of TEOS hydrolysis and further saturation with an antitumor drug. This method allows to increase the efficiency of doxorubicin antitumor drug by 3-4 times compared with free doxorubicin.

The main disadvantage of this method is the unfavorable morphology of boron nitride for cell absorption. Nanotube shaped particles are known to have insufficient activity of cellular uptake and they may be toxic.

**Disclosure of the Invention.** The technical result achieved in the invention is that increasing the efficiency of antitumor chemotherapy by increasing of activity of taking up of nanocontainers with an antitumor drug by cells, prevention of the nanocontainer toxicity for cells due to application of dispersed born nitride nanoparticles 50-300 nm in size with a well-developed outer surface.

Said technical result is achieved in a certain way.

Method of boron nitride nanoparticles fabrication for antitumor drug delivery to tumor cells involves synthesis of spherical boron nitride nanoparticles 50-300 nm in size with a well-developed outer surface by chemical vapor deposition method using reacting ammonia gas, transport argon gas and powder mixtures on the base of amorphous boron and oxygen carrier chemicals.

Chemical deposition is carried out under the following conditions.

$$1000 \le T \le 1430$$

$$1.2 \le \xi \le 8,$$

where T is the temperature in °C of powder mixture, $\xi$ is the ratio of the specific flows $F_{Ar}/F_{NH3}$, wherein $F_{Ar}$ is a specific flow of transport gas and $F_{NH3}$ is a specific flow of reacting gas.

Then using ultrasonication the agglomerates of obtained boron nitride nanoparticles are dispersed, saturated with an antitumor drug by sorption and washed with distilled water.

Boric acid and/or magnesium oxide and/or iron oxide (II) and/or tin oxide (II) and/or their mixtures can be used as an oxygen carrier chemicals.

The content of iron oxide and amorphous boron in the powder mixture is taken based on the following weight ratio, wt. %:

iron oxide 70-91
amorphous boron 9-30

The content of magnesium oxide and amorphous boron in the powder mixture is taken based on the following weight ratio, wt. %:

magnesium oxide 65-84
amorphous boron 16-35

The content of tin oxide and amorphous boron in the powder mixture is taken based on the following weight ratio, wt. %:

tin oxide 75-95
amorphous boron 5-25

The content of boric acid and amorphous boron in the powder mixture is taken based on the following weight ratio, wt. %:

boric acid 85-92 %
amorphous boron 8-15 %

The content of iron oxide, magnesium oxide and amorphous boron in the powder mixture is taken based on the following weight ratio, wt. %:

iron oxide 59-86
magnesium oxide 5-12
amorphous boron 9-32

The content of boric acid, magnesium oxide and amorphous boron in the powder mixture is taken based on the following weight ratio, wt. %:

boric acid 65-91
magnesium oxide 3-10
amorphous boron 6-25

Boron nitride nanoparticles are dispersed using ultrasound method with a power of 40-100 W for 30 minutes.

Sorption of an antitumor drug on boron nitride nanoparticles is perform by continuous agitation of the dispersed nanoparticles in an antitumor drug solution with a concentration of 0.5-5.0 mg/ml for 12-24 h using a magnetic stirrer at a 250 rpm speed.

Sorption of an antitumor drug on boron nitride nanoparticles alternatively can be carried out using ultrasonic treatment of the dispersed nanoparticles in an antitumor drug solution with a concentration of 0.5-5.0 mg/ml with power of 150 W for 15-60 min.

Said antitumor drug is selected from synthetic or natural antitumor drugs.

Said synthetic antitumor drug is selected from alkylating drugs or metabolic antagonists or synthetic drugs of other groups.

Said alkylating synthetic antitumor drug is selected from chloroethylamines or ethyleneamines or nitrosourea derivatives or methanesulfonic acid derivatives.

Said metabolic antagonist synthetic antitumor drug is selected from folic acid antagonists or purine antagonists or pyrimidine antagonists.

Said synthetic antitumor drug of other groups is selected from prospidinum or spirasidine or dicarbasine or natulan or cisplatine or imizadolecarboxamide.

Said natural antitumor drug is selected from antibiotics or phytogenous drugs.

Said natural antitumor drug of the antibiotics group is selected from adriamycin or bleomycin or dactinomycin or rubomycin or bruneomycin or mitomycin C.

Said natural antitumor drug of vegetable origin is selected from colchamine or vinblastine or vincristine.

The boron nitride nanoparticles with a well-developed outer surface are characterized by a hollow spherical central part and multiple petals of exposed BN layers at the side area. The high porosity and the significantly increased specific surface area of the nanoparticles allow boron nitride nanocontainers to retain medical drug molecules and deliver them to tumor cells thus increasing the efficiency of antitumor therapy.

**Embodiments of the Invention.** The invention is implemented as follows.

A powder mixture comprising oxidizing reactants and amorphous boron is prepared. The following reactants are used: boric acid and/or magnesium oxide and/or iron oxide (II) and/or tin oxide (II) and/or mixtures thereof.

The magnesium oxide powder is preliminarily annealed in a muffle or tube electric furnace in air at 400°C.

The powder mixture components are weighed on a laboratory balance with an accuracy of 0.1 mg.

Synthesis of spherical boron nitride nanoparticles is carried out using a high-temperature induction furnace. The crucible containing the powder mixture is placed onto the bottom of the reactor made from boron nitride which is installed inside a graphite heater in the reactor chamber.

The reactor is a demountable cylindrical structure with an outer diameter of 75 mm, an inner diameter of 55 mm and a height of 340 mm. The side wall of the reactor has a 3 mm diameter opening in its upper section for the release of synthesized spherical boron nitride nanoparticles. The graphite heater of the reactor is spaced from the reactor chamber walls with carbon/carbon composite lining. The reactor design provides delivery of the argon transport gas from the bottom upwards and the ammonium reactant gas from the top downwards.

The size and chemical composition of the particles are controlled using a JEOL JSM-7600F electron microscope (JEOL, Japan) equipped with an energy-dispersive X-ray spectroscopy detector.

The average size of the spherical nanoparticles varies between 50 and 300 nm and primarily depends on the synthesis temperature and the flow rates of the transport gas (argon) and the reactant gas (ammonia).

The experimental temperature determines the volume fraction of boron nitride nanoparticles in the synthesis products and their morphology. To obtain boron nitride nanoparticles of the required size and morphology in a quantity of above 80% (by volume) one should carry out the synthesis at temperatures of 1000-1430°C on powder mixture. Below 1000°C the growth rate of boron nitride structures significantly slows down due to the low reaction speed between boron and the oxidizing reactant, while above 1430°C a volume fraction of spherical boron nitride nanoparticles of the required size is decreasing.

The reaction between amorphous boron and the oxidizing reactant produces volatile boron oxides which are carried over by the transport gas (argon) to the ammonia reaction area where the nanostructures synthesis takes place.

The ratio of the flows of argon transport gas and ammonia reaction gas determines the kinetics of boron nitride nanoparticle synthesis and hence the diameter of the nanostructures. High volume fractions of 50-300 nm sized nanoparticles (>80 vol.%) are achieved at the flow ratio $\xi = F_{Ar}/F_{NH3}$ in the range $1.2 \leq \xi \leq 8$.

At gas flow ratios $\xi \leq 1.2$ the ammonia reaches the powder mixture and produces a layer of boron nitride microparticles on its surface which prevents further release of gaseous boron oxide and thus significantly impedes the synthesis of spherical boron nitride nanoparticles with a resultant abrupt decline in a final product output.

At gas flow ratios $\xi \geq 8$ gaseous boron oxide has no enough time for complete reaction with ammonia which also reduces the output of spherical boron nitride nanoparticles and causes the formation of large quantities of solid boron oxide.

Ratio of components in the powder mixture is selected from the abovementioned ranges.

Beyond those ranges, either no spherical boron nitride nanoparticles form, or their size changes, or the quantity of spherical boron nitride nanoparticles of the required size in synthesis products decreases.

Spherical boron nitride nanoparticles with the well-developed outer surface form in a result of the interaction of boron oxide vapors and ammonia within the limited temperature range of (1000-1430°C) at a specific flow ratio of the argon transport gas carrying gaseous boron oxide and the ammonia reaction gas during synthesis.

Spherical boron nitride nanoparticles are often agglomerated in synthesis products. To obtain suspension containing individual boron nitride nanoparticles, one should carry out ultrasonic dispersion at a power of 40-100 W for 30 min in 40 ml of distilled water. The range of ultrasound power of the 40-100 W is chosen because at lower powers the boron nitride nanoparticle agglomerations do not split into individual nanoparticles, whereas higher power causes heating of the suspension which may lead to partial hydrolysis of BN nanoparticles. If ultrasonic treatment is carried out less than 30 minutes the required agglomeration dispersion degree is not achieved, while treatment longer than 30 minutes may destruct the nanoparticles.

For making boron nitride nanoparticles capable of destroying tumor cells they are saturated with an antitumor drug.

Saturation of boron nitride nanoparticles with an antitumor drug is achieved by sorption through continuous agitation of the dispersed nanoparticles in an antitumor drug solution with a concentration of 0.5-5.0 mg/l for 12-24 h using a Heidolph MR (Hei-Standard, Germany) magnetic stirrer at a speed of 250 rpm. Concentrations less than 0.5 mg/ml do not ensure the required therapy efficiency, while at concentrations of greater than 5.0 mg/ml the suspension of BN nanoparticles does not retain stability for more than 24 h.

The time required for the boron nitride nanoparticles saturation with an antitumor drug is at least 12 h. Treatment for less than 12 h does not achieve the limit saturation of the nanoparticles with the drug. Treatment for 12-24 h is sufficient for the limit saturation of the nanoparticle surface with the antitumor drug. Treatment for more than 24 h does not provide any additional sorption of the antitumor drug by the nanoparticles.

An alternative method of saturating the particles with the drug is ultrasonic treatment on a Bandelin Sonoplus HD2200 device, Germany, at 150 W for 15-60 min.

Higher power and longer treatment may destruct the molecules of the antitumor drug, while below these ranges the treatment will not provide the necessary concentration of antitumor drug required in the boron nitride nanoparticles.

Studies of the biocompatibility of boron nitride nanoparticles have shown that the 50-300 nm nanoparticle size is the optimum one from the viewpoint of their absorption by tumor cells. Furthermore the surface of finer particles does not provide for the transportation of the sufficient quantity of drug for efficient destruction of tumor cells, whereas larger particles are not consumed by tumor cells.

Spherical boron nitride nanoparticles with a well-developed outer surface provide the required sorption value of the drug and its subsequent delivery to the tumor cells.

Table 1 shows embodiments of the invention with different synthesis parameters of boron nitride nanoparticles with a well-developed outer surface. Table 2 shows embodiments of the invention with different antitumor drug saturation methods.

**Example 1**

Spherical boron nitride nanoparticles were synthesized by chemical vapor deposition in a vertical induction furnace VIN-1,6-20 (Vac ETO Co., Russia). A crucible with thoroughly homogenized powder mixture (mass was 10.88 g) consist of 59 wt.% iron oxide, 12 wt.% magnesium oxide and 29 wt.% amorphous boron was placed in the furnace. The working chamber of the reactor was heated to 300°C assisted by permanent vacuum pumping for removal of water and oxygen adsorbed on the work chamber surface. Then the chamber was filled with argon to atmospheric pressure. Flows of argon transport gas and ammonia reaction gas were open in the furnace after heating to 850°C. After heating to 1310°C, the temperature was kept constant during 200 min. The working chamber was cooled down to 40-50°C after the synthesis final followed by ventilation with air and opening.

The snow-white fozy powder with masse of 280 mg was received as results of synthesis. According to SEM observations, this snow powder is revealed of agglomerates of boron nitride nanoparticles of 1-5 $\mu$m in diameter. Individual BN nanoparticles with diameter of 100-150 nm had a spherical shape and well-developed outer surface.

Stable suspension of individual boron nitride nanoparticles with a well-developed outer surface was obtained in the ultrasonic disperser "Sonoplus HD2200" (Bandelin, Germany). Ultrasonic treatment during 30 minutes in a 40 ml of distilled water at disperser power of 100 W was carried out due to separate the BN nanoparticles agglomerates. The boron nitride nanoparticles concentration was 2 mg/ml. Study of the particle size distribution after ultrasonic treatment showed that more than 80% of the particles have diameter of 100-200 nm.

Spherical boron nitride nanoparticles were saturated with an antitumor drug by continuous mixing of boron nitride nanoparticles in an antitumor drug solution in a magnetic stirrer "Heidolph MR" (Hei-Standard, Germany). Nanoparticles were separated from the liquid phase using an Eppendorf (Germany) centrifuge. The concentration of the doxorubicin antitumor drug water solution was 0.5 mg/ml. After antitumor drug saturation, boron nitride nanoparticles were triple washed in distilled water and centrifuged for the removal of drug residues from nanoparticles surface.

The absorption of drug saturated nanoparticles by neoplasm cells was studied using confocal microscopy of a culture of Alexa488-falloidine colored cells. The viability of the neoplasm cells after exposure with nanoparticles was studied by measuring the dynamics of transformed cell proliferation during cultivation in the growth media in the presence of drug saturated nanoparticles during 7 days and comparing the results with control samples using fluorescent microscopy control and DAPI staining.

Confocal microscopy showed that boron nitride nanoparticles loaded with doxorubicin are capable to penetrate into transformed (tumor) cells of the IAR-6-1 line. The viability test results showed that inside of tumor cells doxorubicin is released from particles and destructs the cell.

## Example 2

Powder mixture with mass of 11.76 g consist of 78 wt.% boric acid, 4 wt.% magnesium oxide and 18 wt.% amorphous boron was placed in a crucible in the reactor. After preliminary reactor purification from impurities, inert gas filling, heating to the working temperature and the delivery of the transport and reactant gas flows (methodic described in Example 1), the work temperature was established at 1190°C and kept constant during 320 min. The result of synthesis was snow-white fozy powder with mass of 345 mg that were agglomerated of BN nanoparticles with diameter 70-100 nm.

Ultrasonic dispersion was carried out in distilled water at a power of 80 W during 30 min. The concentration of boron nitride nanoparticles was 2 mg/ml. Study of the particle size distribution showed that the quantity of nanoparticles and their agglomerates greater than 250 nm in size was less than 1%.

Boron nitride nanoparticles were saturated with an antitumor drug by ultrasonic treatment of nanoparticles in a 5 mg/ml doxorubicin water solution during 15 min.

Biological tests using transformed (tumor) IAR-6-1 cells in accordance with methodic described in Example 1, showed that the cells successfully absorbed doxorubicin loaded nanoparticles that caused cell death.

**Table 1. Synthesis Parameters of BN Nanoparticles with a Well-Developed Outer Surface**

| № | Powder mixture composition, wt. % | | | | | T, °C | ξ | Spherical BN nanoparticles with a well-developed outer surface | Diameter of spherical BN nanoparticles, nm | Volume fraction of BN nanoparticles in synthesis products, % |
|---|------|-----|----------|-----|----|------|-----|---|---|---|
| | FeOx | MgO | H$_3$BO$_3$ | SnO | B | | | | | |
| Optimal parameters corresponding to the claimed formula | | | | | | | | | | |
| 1 | 59 | 12 | - | - | 29 | 1310 | 2 | + | 80-100 | > 80 |
| 2 | 65 | 3 | - | - | 32 | 1200 | 1.2 | + | 100-120 | > 80 |
| 3 | 86 | 5 | - | - | 9 | 1130 | 6 | + | 100-300 | > 80 |
| 4 | - | 10 | 65 | - | 25 | 1410 | 2 | + | 100-120 | > 80 |
| 5 | - | 5 | 70 | - | 25 | 1410 | 8 | + | 100-120 | > 80 |
| 6 | - | 4 | 78 | - | 18 | 1190 | 6 | + | 50-100 | > 80 |
| 7 | - | 3 | 91 | - | 6 | 1310 | 4 | + | 100-110 | > 80 |
| 8 | 70 | - | - | - | 30 | 1320 | 2 | + | 100-140 | > 80 |
| 9 | 83 | - | - | - | 17 | 1205 | 1.5 | + | 100-200 | > 80 |
| 10 | 91 | - | - | - | 9 | 1000 | 2 | + | 150-300 | > 80 |
| 11 | - | 65 | - | - | 35 | 1350 | 6 | + | 100-120 | > 80 |
| 12 | - | 71 | - | - | 29 | 1430 | 6 | + | 50-100 | > 80 |
| 13 | - | 84 | - | - | 16 | 1430 | 2 | + | 80-100 | > 80 |
| 14 | - | - | - | 95 | 5 | 1320 | 4 | + | 100-120 | > 80 |
| 15 | - | - | - | 87 | 13 | 1245 | 2 | + | 50-200 | > 80 |
| 16 | - | - | - | 75 | 25 | 1410 | 4 | + | 90-110 | > 80 |
| 17 | - | 40 | - | - | 60 | 1430 | 2 | + | 90-110 | > 80 |
| 18 | - | - | 85 | - | 15 | 1270 | 1.5 | + | 100-120 | > 80 |
| 19 | - | - | 89 | - | 11 | 1330 | 2 | + | 100-120 | > 80 |
| 20 | - | - | 92 | - | 8 | 1000 | 1.2 | + | 160-200 | > 80 |

**Table 2. Modes of Nanoparticle Saturation with Antitumor Drug**

| № | Method of BN nanoparticle saturation with antitumor drug | Ultrasonic dispersion | Antitumor drug solution concentration, mg/ml | Treatment time, h | Distilled water washing | Nanoparticle absorption by cells | Therapy effect |
|---|---|---|---|---|---|---|---|
| | | Optimal parameters corresponding to the claimed formula | | | | | |
| 2 | Ultrasonic treatment | + | 0,5 | 0,75 | + | + | + |
| 6 | Ultrasonic treatment | + | 5 | 0,25 | + | + | + |
| 6 | Ultrasonic treatment | + | 2 | 0,25 | + | + | + |
| 6 | Ultrasonic treatment | + | 2 | 1 | + | + | + |
| 1 | Continuous mixing with a magnetic stirrer | + | 0,5 | 12 | + | + | + |
| 1 | Continuous mixing with a magnetic stirrer | + | 5 | 18 | + | + | + |
| 1 | Continuous mixing with a magnetic stirrer | + | 2 | 12 | + | + | + |
| 8 | Continuous mixing with a magnetic stirrer | + | 2 | 24 | + | + | + |

**Claims**

1. Method of boron nitride nanoparticles fabrication for antitumor drug delivery to tumor cells involves synthesis of spherical boron nitride nanoparticles 50-300 nm in size with a well-developed outer surface by chemical vapor deposition method using reacting ammonia gas, transport argon gas and powder mixtures on the base of amorphous boron and oxygen carrier chemicals wherein chemical deposition is carried out under the following conditions:

$$1000 \leq T \leq 1430$$

$$1.2 \leq \xi \leq 8,$$

where T is the temperature in °C of powder mixture, $\xi$ is the ratio of the specific flows $F_{Ar}/F_{NH3}$, wherein $F_{Ar}$ is a specific flow of transport gas and $F_{NH3}$ is a specific flow of reacting gas, followed by the ultrasonic dispersion of agglomerates of obtained boron nitride nanoparticles, saturation with an antitumor drug by sorption and washing in distilled water.

2. Method of Claim 1 wherein oxygen carrier chemicals can be selected from the boric acid and/or magnesium oxide and/or iron oxide (II) and/or tin oxide (II) and/or their mixtures.

3. Method of Claim 2 wherein the content of iron oxide and amorphous boron in the powder mixture is taken based on the following weight ratio, wt. %:

iron oxide 70-91
amorphous boron 9-30

4. Method of Claim 2 wherein the content of magnesium oxide and amorphous boron in the powder mixture is taken based on the following weight ratio, wt. %:

magnesium oxide 65-84
amorphous boron 16-35

5. Method of Claim 2 wherein the content of tin oxide and amorphous boron in the powder mixture is taken based on the following weight ratio, wt. %:

tin oxide 75-95
amorphous boron 5-25

6. Method of Claim 2 wherein the content of boric acid and amorphous boron in the powder mixture is taken based on the following weight ratio, wt. %:

boric acid 85-92 %
amorphous boron 8-15 %

7. Method of Claim 2 wherein the content of iron oxide, magnesium oxide and amorphous boron in the powder mixture is taken based on the following weight ratio, wt. %:

iron oxide 59-86
magnesium oxide 5-12
amorphous boron 9-32

8. Method of Claim 2 wherein the content of boric acid, magnesium oxide and amorphous boron in the powder mixture is taken based on the following weight ratio, wt. %:

boric acid 65-91
magnesium oxide 3-10
amorphous boron 6-25

9. Method of Claim 1 wherein boron nitride nanoparticles are dispersed with ultrasound treatment with a power of 40-100 W for 30 minutes.

10. Method of Claim 1 wherein sorption of an antitumor drug on boron nitride nanoparticles is perform by continuous agitation of the dispersed nanoparticles in an antitumor drug solution with a concentration of 0.5-5.0 mg/ml for 12-24 h using a magnetic stirrer at a 250 rpm speed.

11. Method of Claim 1 wherein sorption of an antitumor drug by boron nitride nanoparticles alternatively can be carried out by ultrasonic treatment of the dispersed nanoparticles in an antitumor drug solution with a concentration of 0.5-5.0 mg/ml with a power of 150 W for 15-60 min.

12. Method of Claim 1 wherein said antitumor drug is selected from synthetic or natural antitumor drugs.

13. Method of Claim 12 wherein said synthetic antitumor drug is selected from alkylating drugs or metabolic antagonists or synthetic drugs of other groups.

14. Method of Claim 13 wherein said alkylating synthetic antitumor drug is selected from chloroethylamines or ethyleneamines or nitrosourea derivatives or methanesulfonic acid derivatives.

15. Method of Claim 13 wherein said metabolic antagonist synthetic antitumor drug is selected from folic acid antagonists or purine antagonists or pyrimidine antagonists.

16. Method of Claim 13 wherein said synthetic antitumor drug of other groups is selected from prospidinum or spirasidine or dicarbasine or natulan or cisplatine or imizadolecarboxamide.

17. Method of Claim 12 wherein said natural antitumor drug is selected from antibiotics or phytogenous drugs.

18. Method of Claim 17 wherein said natural antitumor drug of the antibiotics group is selected from adriamycin or bleomycin or dactinomycin or rubomycin or bruneomycin or mitomycin C.

19. Method of Claim 17 wherein said natural antitumor phytogenous drug is selected from colchamine or vinblastine or vincristine.


**Patentansprüche**

1. Verfahren zur Herstellung von Bornitrid-Nanopartikeln zur Abgabe von Antitumor-Arzneimitteln an Tumorzellen, umfassend die Synthese von kugelförmigen Bornitrid-Nanopartikeln mit einer Größe von 50 bis 300 nm mit einer gut entwickelten äußeren Oberfläche durch ein chemisches Dampfabscheideverfahren unter Verwendung von reaktivem Ammoniakgas, Argon-Transportgas und Pulvergemischen auf der Basis amorpher Bor- und Sauerstoffträgerchemikalien, wobei die chemische Abscheidung unter den folgenden Bedingungen durchgeführt wird:

$$1000 \leq T \leq 1430$$

$$1{,}2 \leq \xi < 8,$$

wobei $T$ die Temperatur in °C des Pulvergemischs ist, $\xi$ das Verhältnis der spezifischen Ströme $F_{Ar}/F_{NH_3}$ ist, wobei $F_{Ar}$ ein spezifischer Transportgasstrom ist und $F_{NH_3}$ ein spezifischer Reaktionsgasstrom ist, gefolgt von der Ultraschalldispersion von Agglomeraten erhaltener Bornitrid-Nanopartikel, Sättigung mit einem Antitumor-Arzneimittel durch Sorption und Waschen in destilliertem Wasser.

2. Verfahren nach Anspruch 1, wobei Sauerstoffträgerchemikalien aus Borsäure und/oder Magnesiumoxid und/oder Eisenoxid (II) und/oder Zinnoxid (II) und/oder deren Gemischen ausgewählt werden können.

3. Verfahren nach Anspruch 2, wobei sich der Gehalt an Eisenoxid und amorphem Bor in dem Pulvergemisch auf der Basis des folgenden Gewichtsverhältnisses in Gew.-% ergibt:

   Eisenoxid 70-91
   amorphes Bor 9-30.

4. Verfahren nach Anspruch 2, wobei sich der Gehalt an Magnesiumoxid und amorphem Bor in dem Pulvergemisch auf der Basis des folgenden Gewichtsverhältnisses in Gew.-% ergibt:

   Magnesiumoxid 65-84
   amorphes Bor 16-35.

5. Verfahren nach Anspruch 2, wobei sich der Gehalt an Zinnoxid und amorphem Bor in dem Pulvergemisch auf der Basis des folgenden Gewichtsverhältnisses in Gew.-% ergibt:

   Zinnoxid 75-95
   amorphes Bor 5-25.

6. Verfahren nach Anspruch 2, wobei sich der Gehalt an Borsäure und amorphem Bor in dem Pulvergemisch auf der Basis des folgenden Gewichtsverhältnisses in Gew.-% ergibt:

   Borsäure 85-92%
   amorphes Bor 8-15%.

**7.** Verfahren nach Anspruch 2, wobei sich der Gehalt an Eisenoxid, Magnesiumoxid und amorphem Bor in dem Pulvergemisch auf der Basis des folgenden Gewichtsverhältnisses in Gew.-% ergibt:

Eisenoxid 59-86
Magnesiumoxid 5-12
amorphes Bor 9-32.

**8.** Verfahren nach Anspruch 2, wobei sich der Gehalt an Borsäure, Magnesiumoxid und amorphem Bor in dem Pulvergemisch auf der Basis des folgenden Gewichtsverhältnisses in Gew.-% ergibt:

Borsäure 65-91
Magnesiumoxid 3-10
amorphes Bor 6-25.

**9.** Verfahren nach Anspruch 1, wobei Bornitrid-Nanopartikel unter Ultraschallbehandlung mit einer Leistung von 40 bis 100 W für 30 Minuten dispergiert werden.

**10.** Verfahren nach Anspruch 1, wobei die Sorption eines Antitumor-Arzneimittels an Bornitrid-Nanopartikel durch kontinuierliches Rühren der dispergierten Nanopartikel in einer Antitumor-Arzneimittel-Lösung mit einer Konzentration von 0,5 bis 5,0 mg/ml für 12 bis 24 Stunden unter Verwendung eines Magnetrührers bei einer Drehzahl von 250 U/min durchgeführt wird.

**11.** Verfahren nach Anspruch 1, wobei die Sorption eines Antitumor-Arzneimittels durch Bornitrid-Nanopartikel alternativ durch Ultraschallbehandlung der dispergierten Nanopartikel in einer Antitumor-Arzneimittellösung mit einer Konzentration von 0,5 bis 5,0 mg/ml mit einer Leistung von 150 W für 15 bis 60 min. durchgeführt werden kann.

**12.** Verfahren nach Anspruch 1, wobei das Antitumor-Arzneimittel aus synthetischen oder natürlichen Antitumor-Arzneimitteln ausgewählt ist.

**13.** Verfahren nach Anspruch 12, wobei das synthetische Antitumor-Arzneimittel aus alkylierenden Arzneimitteln oder metabolischen Antagonisten oder synthetischen Arzneimitteln anderer Gruppen ausgewählt ist.

**14.** Verfahren nach Anspruch 13, wobei das alkylierende synthetische Antitumor-Arzneimittel aus Chlorethylaminen oder Ethylenaminen oder Nitrosoharnstoffderivaten oder Methansulfonsäurederivaten ausgewählt ist.

**15.** Verfahren nach Anspruch 13, wobei das synthetische Antitumor-Arzneimittel der metabolischen Antagonisten aus Folsäure-Antagonisten oder Purin-Antagonisten oder Pyrimidin-Antagonisten ausgewählt ist.

**16.** Verfahren nach Anspruch 13, wobei das synthetische Antitumor-Arzneimittel anderer Gruppen aus Prospidin oder Spirasidin oder Dicarbasin oder Natulan oder Cisplatin oder Imizadolcarboxamid ausgewählt ist.

**17.** Verfahren nach Anspruch 12, wobei das natürliche Antitumor-Arzneimittel aus Antibiotika oder phytogenen Arzneimitteln ausgewählt ist.

**18.** Verfahren nach Anspruch 17, wobei das natürliche Antitumor-Arzneimittel der Antibiotika-Gruppe aus Adriamycin oder Bleomycin oder Dactinomycin oder Rubomycin oder Bruneomycin oder Mitomycin C ausgewählt ist.

**19.** Verfahren nach Anspruch 17, wobei das natürliche Antitumor-Phytogen-Arzneimittel aus Colchamin oder Vinblastin oder Vincristin ausgewählt ist.

**Revendications**

**1.** Procédé de fabrication de nanoparticules de nitrure de bore pour l'administration de médicament antitumoral à des cellules tumorales, incluant la synthèse de nanoparticules sphériques de nitrure de bore d'une taille de 50 à 300 nm avec une surface extérieure largement développée par un procédé de déposition chimique en phase vapeur utilisant la réaction d'ammoniac gazeux, d'argon gazeux de transport et de mélange de poudre à base de bore amorphe et d'agents chimiques porteurs d'oxygène, dans lequel la déposition chimique est effectuée sous les conditions suivantes :

$$1000 \leq T \leq 1430$$

$$1,2 \leq \xi \leq 8,$$

dans lesquelles T est la température en °C du mélange de poudre, $\xi$ est le rapport des débits spécifiques $F_{Ar}/F_{NH3}$, dans lesquels $F_{Ar}$ est un débit spécifique de gaz de transport et $F_{NH3}$ est un débit spécifique de gaz de réaction, suivi par dispersion aux ultrasons des agglomérats de nanoparticules de nitrure de bore obtenues, saturation avec un médicament antitumoral par sorption et lavage dans de l'eau distillée.

2.  Procédé selon la revendication 1, dans lequel les agents chimiques porteurs d'oxygène peuvent être sélectionnés parmi : acide borique et/ou oxyde de magnésium et/ou oxyde de fer (II) et/ou oxyde d'étain (II) et/ou leurs mélanges.

3.  Procédé selon la revendication 2, dans lequel la teneur d'oxyde de fer et de bore amorphe dans le mélange de poudre est prise en se basant sur le rapport pondéral suivant, en pourcentage en poids :

| oxyde de fer | 70-91 |
|---|---|
| bore amorphe | 9-30. |

4.  Procédé selon la revendication 2, dans lequel la teneur d'oxyde de magnésium et de bore amorphe dans le mélange de poudre est pris en se basant sur le rapport pondéral suivant, en pourcentage en poids :

| oxyde de magnésium | 65-84 |
|---|---|
| bore amorphe | 16 à 35. |

5.  Procédé selon la revendication 2, dans lequel la teneur d'oxyde d'étain et de bore amorphe dans le mélange de poudre est prise sur la base du rapport pondéral suivant, en pourcentage en poids :

| oxyde d'étain | 75 à 95 |
|---|---|
| bore amorphe | 5 à 25. |

6.  Procédé selon la revendication 2, dans lequel la teneur d'acide borique et de bore amorphe dans le mélange de poudre est prise sur la base du rapport pondéral suivant, en pourcentage en poids :

| acide borique | 85 à 92 |
|---|---|
| bore amorphe | 8 à 15. |

7.  Procédé selon la revendication 2, dans lequel la teneur d'oxyde de fer, d'oxyde de magnésium et de bore amorphe dans le mélange de poudre est prise sur la base du rapport pondéral suivant, en pourcentage en poids :

| oxyde de fer | 59 à 86 |
|---|---|
| oxyde de magnésium | 5 à 12 |
| bore amorphe | 9 à 32. |

8.  Procédé selon la revendication 2, dans lequel la teneur d'acide borique, d'oxyde de magnésium et de bore amorphe dans le mélange de poudre est prise sur la base du rapport pondéral suivant, en pourcentage en poids :

| acide borique | 65 à 91 |
| oxyde de magnésium | 3 à 10 |
| bore amorphe | 6 à 25. |

9. Procédé selon la revendication 1, dans lequel les nanoparticules de nitrure de bore sont dispersées par traitement aux ultrasons avec une puissance de 40 à 100 W pendant 30 minutes.

10. Procédé selon la revendication 1, dans lequel la sorption d'un médicament antitumoral sur les nanoparticules de nitrure de bore est effectuée par agitation continue des nanoparticules dispersées dans une solution de médicament antitumoral avec une concentration de 0,5 à 5,0 mg/ml pendant 12 à 24 heures en utilisant un agitateur magnétique à une vitesse de 250 tpm.

11. Procédé selon la revendication 1, dans lequel la sorption d'un médicament antitumoral par les nanoparticules de nitrure de bore peut en variante être effectuée par traitement aux ultrasons des nanoparticules dispersées dans une solution de médicament antitumoral avec une concentration de 0,5 à 5,0 mg/ml avec une puissance de 150 W pendant 15 à 60 minutes.

12. Procédé selon la revendication 1, dans lequel ledit médicament antitumoral est sélectionné parmi des médicaments antitumoraux synthétiques ou naturels.

13. Procédé selon la revendication 12, dans lequel ledit médicament antitumoral synthétique est sélectionné parmi des médicaments alkylants ou des antagonistes métaboliques ou des médicaments synthétiques d'autres groupes.

14. Procédé selon la revendication 13, dans lequel ledit médicament antitumoral synthétique alkylant est sélectionné parmi des chloroéthylamines ou éthylèneamines ou des dérivés nitrosourés ou des dérivés d'acide méthane sulfonique.

15. Procédé selon la revendication 13, dans lequel ledit médicament antitumoral synthétique antagoniste métabolique est sélectionné parmi des antagonistes d'acide folique ou des antagonistes de purine ou des antagonistes de pyrimidine.

16. Procédé selon la revendication 13, dans lequel ledit médicament antitumoral synthétique d'autres groupes est sélectionné parmi : prospidinum, ou spirasidine ou dicarbasine ou natulan ou cisplatine ou imizadolecarboxamide.

17. Procédé selon la revendication 12, dans lequel ledit médicament antitumoral naturel est sélectionné parmi les antibiotiques ou les médicaments phytogènes.

18. Procédé selon la revendication 17, dans lequel ledit médicament antitumoral naturel du groupe des antibiotiques est sélectionné parmi : adriamycine ou bléomycine ou dactinomycine ou rubomycine ou brunéomycine, ou mitomycine C.

19. Procédé selon la revendication 17, dans lequel ledit médicament phytogène antitumoral naturel est sélectionné parmi : colchamine ou vinblastine ou vincristine.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20110033707 A
- WO 2014124329 A
- US 20140147508 A
- US 20130331764 A

**Non-patent literature cited in the description**

- **X. LI ; CH. ZHI ; N. HANAGATA ; M. YAMAGUCHI ; Y. BANDO ; D. GOLBERG.** Boron Nitride Nanotubes Functionalized with Mesoporous Silica for Intracellular Delivery of Chemotherapy Drugs. *Chem. Commun.*, 2013, vol. 49, 7337